# EUROPEAN PATENT SPECIFICATION

(11) **EP 2 953 547 B1**
(45) Date of publication and mention of the grant of the patent: **03.04.2019**
(21) Application number: 14749674.9
(22) Date of filing: 04.02.2014
(51) Int. Cl.: A61B 8/00, A61B 8/12, A61B 8/08, A61M 25/01, A61B 90/00

(54) **NON-LINEAR ECHOGENIC MARKERS**
NICHTLINEARE ECHOGENE MARKER
MARQUEURS ÉCHOGÈNES NON LINÉAIRES

(30) Priority: 05.02.2013 US 201361760899 P
(43) Date of publication of application: 16.12.2015
(73) Proprietor: Muffin Incorporated, West Lafayette, IN 47906 (US)
(72) Inventor: MCKINNIS, Peter, S., West Lafayette, IN 47906 (US); ZHOU, Yun, West Lafayette, IN 47906 (US); FEARNOT, Neal, E., West Lafayette, IN 47906 (US)
(74) Representative: Simpson, Tobias Rutger
(86) International application number: PCT/US2014/014532
(87) International publication number: WO 2014/123834

(56) References cited:
- EP-A1- 0 624 342
- WO-A1-2011/154782
- US-A- 5 201 314
- US-A- 5 327 891
- US-A- 5 759 154
- US-A1- 2002 188 195
- US-A1- 2003 040 756
- US-A1- 2003 135 117
- US-A1- 2004 260 269
- US-A1- 2006 247 530
- US-A1- 2008 097 213

## Description

### BACKGROUND

The present invention relates to echogenically enhanced markers which are used for imaging medical devices within a body.

It is necessary in some medical procedures to place a medical device subcutaneously within a body and then to be able to subsequently and accurately locate the item within the body. These applications have particular importance regarding accurately locating the position of catheters within a body via transcutaneous ultrasound. These applications are also useful for locating feeding tubes, chest tubes, and drainage tubes which are prone to movement within a body. Additionally, many medical procedures require precise positioning of medical devices relative to other body parts and organs such as biopsy procedures, for example.

Current procedures can utilize periodic x-rays in order to locate medical devices positioned subcutaneously. However, x-ray procedures require moving the patient from a bed or room to the x-ray machine. Additionally, x-ray machines can be costly to operate and x-ray procedures are radiation intensive which can cause added complications for a medical patient.

In some procedures, ultrasound imaging is used for imaging medical devices within a body. By applying echogenic markers to medical devices placed within a body, screening processes can be performed bedside by using non-ionizing radiation, e.g. ultrasound waves. This prevents the problems associated with relocating a patient as well as problems associated with extended ionizing radiation exposure which can occur when using x-ray.

However, problems can arise when using ultrasound to image a medical device within a body as ultrasound images are inherently noisy. When using ultrasound images, it can be difficult to precisely locate a medical device especially in relation to body parts and organs due to the inherent lack of clarity with ultrasound imaging. In some cases, standard echogenic markers (such as echotipping) can increase the echogencity of a medical device. However, these devices can still suffer from problems with image noise and the ability of a physician to differentiate between the medical device and body tissue due to the image noise. Additionally, often ultrasound image clarity is heavily reliant on a physician's ability to precisely position the ultrasound transducer relative to the medical device, which can cause increased difficulty with obtaining useable images.

Thus, there is a need for improvement in the field particularly related to echogenically enhanced medical devices or medical devices having echogenic markers which provide distinctive images which enable physicians to precisely position a medical device within the body relative to other medical devices or body tissue.

Reference is directed to US 5,327,891 which discloses catheters having a plurality of vanes external to a central core serving as channels for microbubbles. A track may be provided having grooves in which one of the vanes passes, which track provides additional microbubbles, so as to allow for following the movement of the catheter and track by ultrasonic imaging. The catheter provides for reduced abrasion, as well as ease of following the movement of the catheter through the patient's vessels.

Reference is further directed to EP 0 624 342 which discloses a medical instrument with enhanced ultrasonic visibility. The medical instrument includes an elongate probe or needle for insertion into a patient. Selected locations along the instrument are provided with deposits of a material exhibiting a high degree of ultrasonic reflectivity. The material includes a matrix of gas bubbles contained in a polymeric material. The gas bubbles exhibit high reflectivity of ultrasonar energy.

Reference is further directed to US 5,759,154 which discloses an echogenically enhanced medical device and method for manufacture. A mask is applied to the surface of the medical device, and portions of bare metal are exposed through the mask. The masked device is subjected to a process which removes material from the exposed portions. The resulting depressions enhance echogenicity of the device under ultrasound. In a preferred embodiment the depressions comprise alternating rows of squares and diamonds disposed around the circumference of the device.

Reference is further directed to US 2008/0097213 which discloses medical devices made visible under ultrasonic or magnetic imaging techniques by adding a series of features on their surfaces. The features are desirably placed at more than one angle to the surface in order to enhance the visibility of the surface. Laser-machining can make a series of depressions or voids that are symmetric with respect to the surface and another series of depressions or voids that are non-symmetric. The pattern of voids is also varied by using more than one size of void, the depth of the voids, and the distribution of voids, i.e. more voids in some areas than others.

Reference is further directed to US 2004/0260269 which discloses a method for improving the ultrasonic visibility of medical devices. The method comprises coating with a heavy metal such as gold, rhodium, and platinum. Also provided is an injection needle, the ultrasonic visibility of which is increased by coating at least a portion thereof with a heavy metal.

### SUMMARY

Among other things, disclosed herein are embodiments of medical devices which provide echogenically enhanced imaging visibility when imaged by physicians during ultrasound procedures. In one example, a cylindrical object such as a catheter has an echogenic region with subregions having enhanced echogenicity which are interleaved with subregions that are echolucent compared to the echogenic regions.

The echogenic subregions can include features which make the subregions more echogenic. The echolucent subregions are generally constructed of materials which are echolucent when positioned with any body and surrounded by body fluids and body tissues. The echogenic subregions have features or materials which have acoustic impedance much different than that of body tissues and body fluids.

The echolucent subregions are generally constructed of a polymer or plastic which has acoustic impedance similar to that of body tissues and fluids. Embodiments of a cylindrical medical device have echogenic subregions arranged in a checkerboard pattern such that the echogenic subregions have sharp boundaries and a heterogeneous structure which is capable of producing ultrasound images with characteristically exogenous features easily differentiable from tissue. The echogenic subregions can be constructed in particular examples by printing metal on a polymer structure or surface of a medical device.

Additionally, the echogenic subregions can be included within a plastic structure by forming gaseous pits within the structure. The gaseous pits can be formed by laser pitting, e.g. where a laser is applied to a wall or surface to form gaseous pits beneath the surface. The echogenic subregions can also be created by casting a metal object which is configured to fit within a recess in a plastic structure in such a way that a distinguishable pattern is formed between the cast metal part and the plastic structure. A variety of echogenic materials can be used within the echogenic subregions, such as gases, metals, glasses medium and high density polymers, air bubbles or air pockets.

In an alternative example, a medical device includes a marker or echogenic region which includes a fluid contrast agent. The fluid contrast agent includes microbubbles which are configured to receive a linear ultrasound signal and reflect a harmonic ultrasound signal. The medical devices have a support structure with compartments which are integral to the structure of the medical device. The fluid contrast agent is enclosed within the compartments either permanently or semi-permanently in either an open or closed configuration. The medical devices include multiple compartments positioned within the medical device which are arranged in a variety of different arrangements. During an ultrasound imaging procedure, an ultrasound imaging system can be used in a harmonic imaging mode such as pulse inversion or power modulation. The microbubbles are capable of transforming a reflected signal from a linear signal into non-linear harmonic signals which are distinguishable from signals reflected from body tissue which are generally linear. In this way, the microbubbles can produce a distinct image with a high signal to noise ratio such that a physician can readily identify, locate and position a medical device within a body. The compartments can be configured within a medical device in a variety of different configurations.

Another example includes microbubbles positioned directly within a plastic body or structure that is made of a compliant material. This configuration eliminates the need for a fluid contrast agent to carry or hold microbubbles.

Another example includes a structure or echogenic region which has small resonators designed with a specific resonant frequency and a relatively high Q-factor (i.e. a lower rate of energy loss relative to the stored energy of an oscillator). The resonators are designed with bars that are attached at one end and are configured to resonate and oscillate upon receiving a specific ultrasound signal. The oscillating bars produce a characteristically non-linear response signal which is imageable through certain imaging modes of ultrasound imaging systems, such as pulse inversion and power modulation. The resonators are designed with a high Q-factor in order to increase the signal by reducing the energy loss relative to the stored energy in the resonator such that the oscillations die out more slowly.

Further forms, objects, features, aspects, benefits, advantages, and embodiments of the present disclosure will become apparent from a detailed description and drawings provided herewith.

### BRIEF DESCRIPTION OF THE DRAWINGS

FIG. 1 is a partial perspective view of a medical device having echogenic subregions.
FIG. 2 is a partial perspective view of an alterative medical device having fluid contrast agent compartments.
FIG. 3 is a perspective view of a medical device having resonators.
FIG. 4 is a schematic representation of an alternative configuration of echogenic subregions.

### DESCRIPTION OF THE SELECTED EMBODIMENTS

For the purpose of promoting an understanding of the principles of the disclosure, reference will now be made to the embodiments illustrated in the drawings and specific language will be used to describe the same. It will nevertheless be understood that no limitation of the scope of the claims is thereby intended. Any alterations and further modifications in the described embodiments, and any further applications of the principles of the disclosure as described herein are contemplated as would normally occur to one skilled in the art to which the invention relates. Particular embodiments are shown, although it will be apparent to those skilled in the relevant art that some features that are not relevant to the present disclosure may not be shown for the sake of clarity.

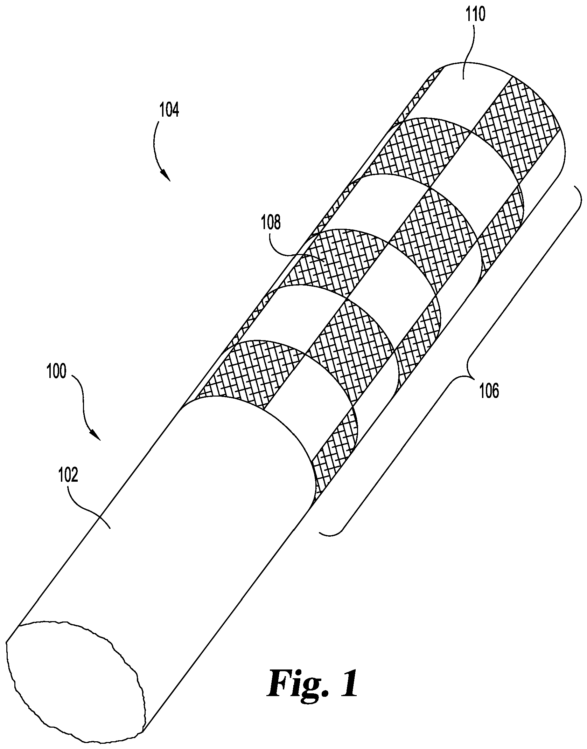

A medical device 100 is depicted in Fig. 1. The medical device 100 is configured to be used in conjunction with an ultrasound imaging system. More particularly, the medical device 100 is configured to be used with any of a variety of ultrasound imaging systems which are commonly used for medical procedures and applications. In that way, the medical device 100 is configured to work in conjunction with existing ultrasound consoles, probes and pulse sequences used within a variety of ultrasound imaging systems.

The medical device 100 is representative of medical devices which are commonly used for subcutaneous medical procedures, such as catheters, guidewires, and biopsy needles. Alternatively, the medical device 100 can be a separate component that may be installed or retrofitted with such medical devices. The medical device 100 is configured to be positioned in a permanent or semi-permanent state within a body. The medical device 100 is generally cylindrically shaped in the illustrated embodiment, with an outer surface 102 and an end 104. The end 104 is representative of a tip or portion of a medical device such as a catheter tip or biopsy needle tip. The medical device 100 can include one or more lumens positioned internally.

Generally an ultrasound signal is partially reflected at the interface of two mediums having different acoustic impedances such as water or body tissue and a metal surface. During ultrasound procedures, a transducer emits an ultrasound signal and the signal is reflected partially as it encounters changes in the medium through which it travels. A portion of the reflected signal returns to a transducer. The signal is processed to create an image viewable by a physician. The characteristic ability of an object to reflect ultrasound waves is described herein as "echogenicity". The term "echolucent" is used herein to describe a characteristic of an object, wherein that object generally reflects less ultrasonic signal in comparison to a second object in the context of a particular environment. In other words, "echolucency" describes the characteristic of having less acoustic attenuation in comparison to something else in a particular environment.

Positioned at the end 104 is an echogenic region 106. The echogenic region 106 includes echogenic subregions 108 and echolucent subregions 110. The echogenic subregions 108 are configured to be echogenic when placed within a body and among body tissues and fluids. The echolucent subregions 110 are configured to be echolucent when placed within a body and among body tissues and fluids.

In some embodiments, the echogenic subregions 108 and the echolucent subregions 110 are arranged in an alternating, discontinuous or broken pattern on the medical device 100. As an example, the echogenic subregions 108 and the echolucent subregions 110 are arranged in a checkerboard pattern (FIG. 1). The checkerboard pattern in this embodiment is configured in such a way that the echogenic subregions 108 each have borders (shown as straight lines or sides in a particular example) which are shared with nearby subregions 108 generally at a corner point, but are otherwise isolated and not shared with any neighboring echogenic subregions 108. In that way, the borders of each echogenic subregion 108 are geometrically independent of those of the other echogenic subregions 108. The checkerboard pattern is positioned on the outer surface 102 such that it wraps around the cylindrical outer surface 102 of the medical device 100 in the echogenic region 106. The term "border" as used herein includes borders creating discrete subregions as well as border areas having a gradual change between echogenic subregions and echolucent subregions. For example, the borders can indicate a sinusoidal echogenic difference between subregions.

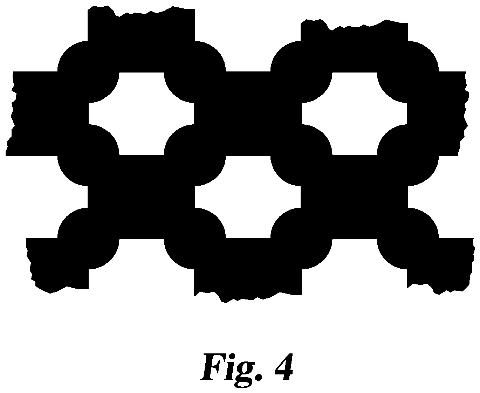

In some embodiments, the echogenic subregions are continuous while the echolucent subregions are discontinuous. In other embodiments, the echogenic subregions are discontinuous while the echo lucent subregions are continuous. One example of such arrangements is shown in FIG. 4. In all cases, the echogenic subregions and the echolucent subregions are configured in a pattern or arrangement that when visualized on screen is readily distinguishable from body tissue.

The medical device 100 is constructed largely of a plastic or polymer material having acoustic impedance similar to that of body tissue and body fluids. Because of this, ultrasound signals travelling through body tissue and encountering the polymer surface of the medical device 100 will mostly continue along their original trajectory with a minimal portion of the signal being reflected. In this way, the polymer makeup of the echolucent subregions 110 causes the echolucent subregions 110 to be echolucent when placed within a body and imaged through ultrasound imaging procedures.

The echogenic subregions 108 include echogenic enhancements. The echogenic subregions 108 can be configured and produced in a variety of different ways. In one example, the medical device 100 can be structured from a plastic material and the echogenic subregions 108 can include a metal film which is printed on, laid over or otherwise placed on the plastic outer surface 102 of the medical device 100. In that example, the echolucent subregions 110 include the polymer material which makes up the structure of the medical device 100.

The metal material added to the polymer structure in the checkerboard pattern as described herein has acoustic impedance much greater than most polymers, body tissue, and body fluids. The difference between the acoustic impedance causes the metal to be echogenic when placed within a body. The echogenic subregions 108 will reflect an ultrasound signal when positioned within a substance such as water or body tissue and when used during a procedure involving ultrasound imaging equipment.

The medical device 100 can additionally be a marker which is configured to be used with commercially available subcutaneous products such as catheters or biopsy needles. In this way a catheter or biopsy needle can be retrofitted with a medical device as described herein. For example, the medical device 100 can be positioned within a lumen of a catheter or alternatively it can be attached externally to the catheter.

When performing an ultrasound procedure using a subcutaneously placed medical device 100, a transducer which emits ultrasound signals is applied (e.g. against the patient's body) so that its signals are directed toward medical device 100. At least a portion of the ultrasound signals hit the medical device 100 and a portion of the signals are reflected back towards the transducer. The amount of reflected signal is generally dependent on the relative angle between the transducer face and the surface 102. When the major axis of the medical device 100 is positioned perpendicular to the direction of the transducer's signals (e.g. substantially parallel with the transducer face), a first portion of the ultrasound signal reflects from a portion of the medical device 100 (i.e. multiple subregions 108) to the transducer face. The reflecting portion of the medical device 100 is that portion which is significantly echogenic and has a surface normal which is parallel to the transducer face. Similarly, a second portion of the ultrasound signal reflects from portions of the medical device 100 which do not have a surface normal which is parallel to the transducer face (e.g. subregions 108 on a portion of medical device 100 generally oblique with respect to the transducer). The second portion of the ultrasound signal is reflected away from the transducer face to a greater degree than is the first portion of the ultrasound signal. The reflecting portion of the medical device 100 thus appears in an ultrasound image as a generally straight line in an axial direction relative to the medical device 100. Due to the checkerboard pattern of the echogenic subregions 108 and the echo lucent subregions 110, the visually imaged reflecting portion appears as a broken or dotted line.

The reflecting portion of the medical device 100 can be enlarged or enhanced by providing further enhancements to the echogenic subregions 108. For example, a texturing can be applied to part or all of a metal surface forming (or which will form) one or more subregions 108. Such texturing may be or include small cavities that are machined, etched or otherwise textured into the surface of the echegenic regions 108, such as dimples, divots, grooves, lines, or ridges. With enhancements to the echogenic subregions 108, the portion of the ultrasound signals which reflect back to the transducer can reflect from a greater surface area of the medical device 100. In that way, the clarity of the resulting image is less dependent on the relative positions of the transducer face and the reflecting portion of the medical device 100, as the echogenic subregions 108 will reflect ultrasound signals in a wider range of directions. This configuration additionally allows the ultrasound imaging system to visually represent more distinctly the exogenous features of the echogenic subregions 108. For example, the sharp corners of the echogenic regions 108 can be more visible as the image will encompass more than simply a dotted line.

The visual appearance of distinct dotted lines and sharp corners during the ultrasound imaging procedure create a contrast which is readily observable by a physician. The echogenic region 106 enhances the visibility of the medical device during ultrasound imaging procedures by producing a reflection with a signal pattern that is not produced by body tissue and which is readily differentiable from tissue. The checkerboard pattern of the echogenic subregions 108 and echo lucent subregions 110 makes the echogenic region 106 contrasting with the surrounding body tissue under imaging. Structures and organs in a body tend to have rounded boundaries and produce a homogenous echo. In some embodiments, the echogenic subregions 108 have sharp corners, straight boundaries or borderlines and a relatively heterogenic structure in the illustrated embodiment, which allows the echogenic region 106 to produce images with characteristic features which are readily differentiable from body tissue. When imaged using an ultrasound imaging system, the sharp corners of subregions 108 are visually distinct from surrounding body tissue, allowing a physician to more easily and readily identify the position of the medical device 100.

The medical devices described herein are useful for positioning catheters via transcutaneous ultrasound. By positioning an echogenic region 106 on a catheter or other medical device as such as described above, a physician can readily identify the location of a medical device relative to body tissues and organs. In this way, the physician can be confident in the accuracy of the physician's assessment of the location of the medical device by viewing the distinctive features or echogenic subregions positioned within the echogenic region. The devices discussed herein can also be advantageous for use in monitoring feeding tubes, chest tubes and drainage tubes which are prone to movement.

The echogenic subregions 108 are sized for effective placement on device 100 as well as for effective reflection of ultrasound waves. If the echogenic subregions 108 are too large, the echogenic region 106 may be difficult or impossible to include on certain medical devices 100 where size is a critical feature. Alternatively, if the echogenic subregions 108 are too small it will be difficult for a physician to visually distinguish the echogenic subregions 108 from noise which is inherently part of an ultrasound image. It has been determined that an optimal size for the echogenic subregions 108 is about 1 to 1.5 times the resolution of the imaging machine or the resolution of the ultrasound signal. It has further been determined that for a typical 6 MHz ultrasound signal, echogenic subregions 108 in the range of 0.05 mm to 2.5 mm (e.g. on a side in the square checkerboard embodiment of Fig. 1) are suitable. It is also a feature of the present disclosure that the echogenic region 106 is several millimeters large due to multiple echogenic subregions 108 positioned within the echogenic region 106.

The medical devices described herein can be constructed in a variety of different shapes and dimensions. For example, medical devices can be constructed as planar structures having substantially flat planar surfaces. The medical device can have one or more flat planar surfaces which can include an echogenic region. Additionally, the medical devices can have a structure which is more rounded or cylindrically shaped, a type of which the medical device 100 of Fig. 1 is but one example. The medical devices described herein can have a variety of different echogenic regions including various types of echogenic subregions and echolucent subregions. For example, the medical device 100 includes a checkerboard pattern; however, medical devices can include echogenic regions which have a series of parallel stripes or alternatively a series of angled stripes or zigzagging stripes.

In other embodiments, a medical device 100 can have regions 106 with echogenic subregions 108 that, when viewed together, form letters or text positioned within the echogenic region 106. For example, square or other subregions 108 may be placed with respect to each other, with echolucent subregions 110 adjacent them, to provide a letter, symbol or word, e.g. indicating the side or orientation being viewed. Thus, during ultrasound imaging procedures, the letters reflect ultrasound signals which when imaged form a distinct symbol or term readily identifiable by or leaping to the attention of a physician or other viewer.

The echogenic regions described herein can include echogenic subregions or features which include any of a variety of different echogenic enhancements. Examples include gas bubbles or pits, glass beads, metals or metal particles, sandblasted surfaces, laser treated surfaces, medium density polymers, or high density polymers. Additionally, the echolucent subregions located within the echogenic regions described herein can be constructed of materials having acoustic impedance similar to that of body tissue and fluids such as low density polymers, for example polyurethane.

The echogenic subregions described herein can be constructed in a variety of different ways. For example, a metal film can be printed on a plastic surface whereby the metal surface when compared to the plastic surface and when positioned within a body having body fluids and body tissues will be echogenic compared to the plastic surface and the body tissues due to the greater acoustic impedance of the metal surface.

As noted, the echogenic subregions or features can include a plastic structure which has gaseous pits formed beneath the surface of the structure. In this example, the medical device includes a plastic surface and gaseous cavities or bubbles located beneath the surface yet within the plastic material. In the case of a catheter, for instance, an embodiment can include gaseous pits positioned between an inner surface and an outer surface of a catheter wall. The gaseous pits are formed by laser pitting, i.e. applying a laser to the plastic of the wall to form a bubble or pit in the wall. The gaseous pits are generally spherical or otherwise include curved surfaces in particular embodiments.

Gaseous pits as described herein include a gas enclosed within a plastic structure. The gas has acoustic impedance which is different from the plastic structure. The difference in acoustic impedance causes a portion of an ultrasound signal to be reflected upon encountering a boundary between a gaseous pit and the plastic structure. A gaseous pit having a curved shape boundary results in an ultrasound wave reflecting in a range of directions, with at least a portion of the reflected signal returning to the imaging transducer. In this way, medical devices having echogenic subregions including gaseous pits are imageable at a variety of viewing angles. Similar to the medical device 100 described above, the gaseous pits are included within defined echogenic subregions 108, e.g. squares in a checkerboard pattern, or areas arranged to form a symbol or word.

As one illustration, a medical device can be constructed by casting a specific plastic shape and a specific metal shape which are then fitted together. In the case of the medical device 100 discussed above, a plastic structure can be cast or molded to have recesses. The metal sections are cast and fit into the recesses in a pattern such as the checkerboard pattern, in which the metal sections each have isolated borders. A variety of different physical structures and shapes can be achieved in this way.

In another example, a plastic portion can be cast having recesses which are configured to accept echogenic subregions in the form of a checkerboard or other arrangement as described previously. The echogenic subregions are created by casting or molding plastic shapes which are configured to fit within the recesses of the plastic portion. Gaseous pits are formed within the echogenic subregions by laser pitting parts of the echogenic subregions. Once inserted into the recesses, the echogenic subregions form a pattern which is visually identifiable during ultrasound imaging procedures.

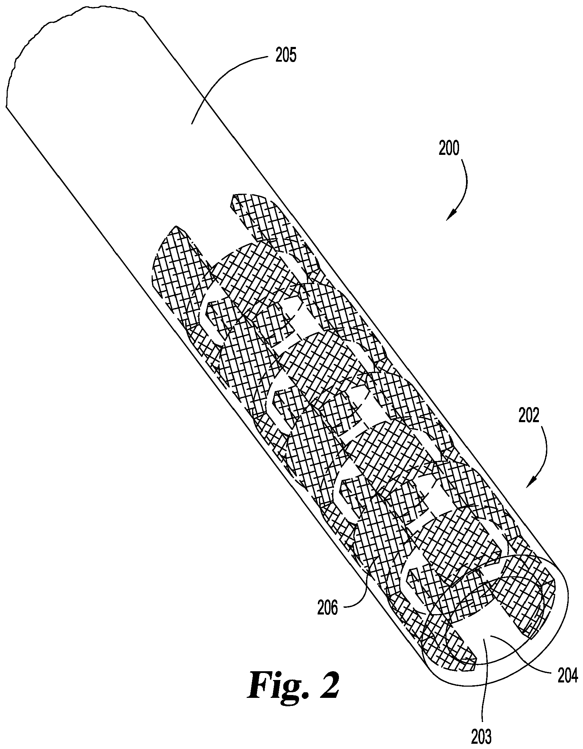

Another example of a medical device 202 (e.g. Fig. 2) includes an echogenic region having a fluid contrast agent. The fluid contrast agent is a fluid which is echogenic compared to a surrounding environment and in particular when compared to body fluids and body tissue. Device 200 includes a portion 202 (e.g. a tip or medial portion or sleeve). In a particular example, device 200 or portion 202 can be all or part of a catheter. In other examples, device 200 or portion 202 could be part or all of other devices place in the body, as noted previously. Portion 202 is an end portion in the illustrated embodiment that includes a lumen (defined by an inner surface 204) and an outer surface 205. Positioned between inner surface 204 and outer surface 205 (i.e. within the wall of portion 202) are cavities, compartments, or capsules 206.

Portion 202 is of a material that is generally echolucent. Capsules 206 are filled with a fluid contrast agent having echogenic properties. The capsules 206 in the Fig. 2 example are integral with (e.g. formed within) portion 202 of medical device 200 and a fluid contrast agent (represented by the hatching or texturing in Fig. 2) is enclosed in the capsules 206. The capsules 206 can be sealed in a closed configuration or alternatively they can be accessible whereby the fluid contrast agent can be introduced into and/or removed from capsules 206, permitting the agent to be replaced or introduced in a particular desired fashion or to produce a particular desired configuration.

The fluid contrast agent can include a variety of echogenic materials, one of which is microbubbles. Generally, microbubbles include a gas core and a shell. The shell material is preferably elastic. The elasticity of the shell material affects the echo reflectivity of the microbubble. The more elastic the material, the more acoustic energy it can withstand before bursting. The microbubble shell can be composed of certain materials such as albumen, galactose, lipid or polymers.

Such microbubbles can be advantageous for producing an ultrasound image with increased clarity, allowing the physician or other viewer to be able to accurately locate and position a medical device within a body. Microbubbles have a characteristic such that when a linear ultrasound signal intercepts a microbubble, the microbubble reflects a non-linear harmonic signal. The harmonic signal is distinct from body tissue in that body tissue does not inherently echo harmonic signals. When a microbubble reflects an ultrasound signal, the harmonic components are generated at the moment the signal is scattered. Thus, the microbubbles do not depend on any preexisting harmonic component in the initial ultrasound signal. In this way, microbubbles are capable of generating a harmonic signal from a linear input signal. Body tissue, on the other hand, generally reflects harmonic signals only when a harmonic signal is first emitted from the transducer. This result can be used advantageously for producing images with greater clarity and signal to noise ratio, insofar as a linear ultrasound signal from the transducer provides harmonic reflection from a device 200 and a differentiation from waves reflected by tissue. In this way, microbubbles strategically positioned within a medical device provide enhanced visibility of that medical device when placed within a body and used with an ultrasound imaging system.

Ultrasound imaging systems commonly have methods of detecting harmonic signal reflections using methods such as pulse inversion and power modulation. Ultrasound imaging systems having these types of modes are capable of emitting a linear ultrasound signal and then detecting a non-linear harmonic ultrasound signal. Because body tissues do not inherently echo harmonic signals, ultrasound imaging systems operating in a harmonic mode do not image body tissues with the same clarity as they do with respect to microbubbles that reflect harmonic signals. In this way, an image with high signal to noise ratio can be obtained which shows the medical device with particular clarity relative to body tissues.

The capsules 206 can function in a similar manner as the echogenic subregions described previously. For example, the capsules 206 shown in the Fig. 2 diagram are arranged in a checkerboard pattern. However, the capsules 206 are not limited to the configuration shown in Fig. 2 and can be formed in a variety of shapes and positioned in a variety of arrangements. For example, the capsules 206 can be shaped as lumens or channels (e.g. made during extrusion of device 200 and/or portion 202) which run through the wall of device 200 and/or portion 202, e.g. circumferentially around and/or longitudinally along or within the lumen. The capsules 206 can be a number of individual capsules positioned randomly within the medical device 200, as another example. A single capsule 206 placed within the medical device 200 may be provided.

As another example, microbubbles can individually be positioned within a plastic body or portion of a medical device. The microbubbles can be small or sparse with a space of several multiples of the microbubbles' diameters separating each microbubble, and the device's plastic or other material can be relatively compliant such that the boundary of each microbubble is sufficiently elastic to be able to reflect an ultrasound signal and produce a harmonic signal. In such a configuration, a medical device can have echogenic subregions having microbubbles positioned directly within the structure. The medical device can simultaneously have echolucent subregions where no microbubbles are located such that a pattern is created by the echolucent subregions and the echogenic subregions.

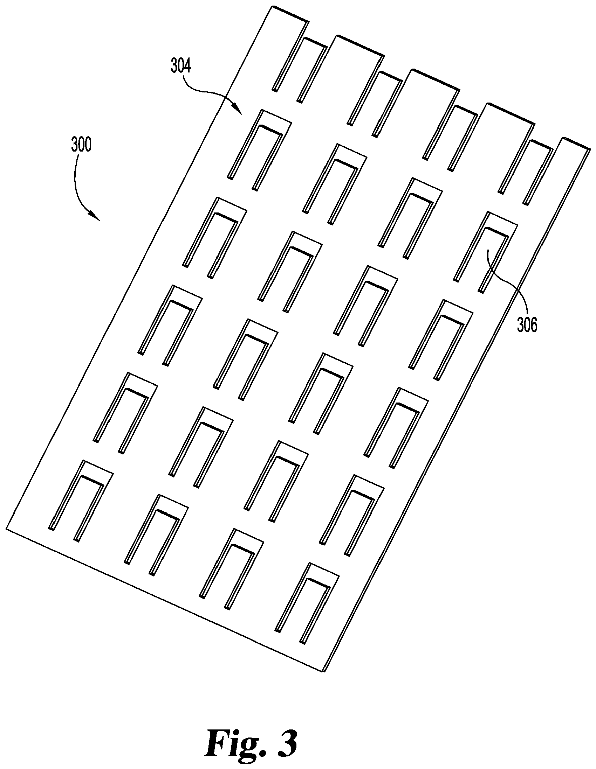

In an alternative example shown in Fig. 3, a medical device or marker 300 includes a base 302 with a plurality of resonators 304 designed with a specific resonant frequency and a relatively high Q-factor. The illustrated embodiment of resonators 304 is an elongated bar or tongue 306 that is attached at one end to part of base 302 of medical device or marker 300. The bar 306 is configured to oscillate at a specific natural frequency. During ultrasound imaging procedures, an ultrasound signal emitted at the natural frequency causes the bar 306 to oscillate in such a way that an enhanced harmonic signal is returned to an ultrasound imaging transducer. The Q-factor of resonators 304 is high so that resonators 304 have a minimal rate of energy loss relative to the stored energy in the resonator 304. In other words, the quality factor is high so that the oscillations will die out more slowly. The resonant frequencies emitted by the bars 306 are received by the ultrasound imaging system operating in a specific mode configured to receive harmonic signals (such as pulse inversion or power modulation modes). In that way, the non-linear signals produced by the resonators 304 during an ultrasound imaging procedure provide an enhanced image with a high signal to noise ratio thereby enhancing a physician's ability to locate and position a medical device including resonators 302 or a marker positioned within a medical device having resonators 302. A medical device may be fashioned itself with a portion (e.g. a part of an outside surface) configured as discussed above, or a marker or separate piece configured as discussed above may be inserted into, formed into, attached to or otherwise associated with a medical device, for enhanced reflection as indicated.

The medical devices described herein are representative of medical devices which are commonly used for subcutaneous medical procedures, such as catheters, guidewires, and biopsy needles. Alternatively, the medical devices can be a separate component that may be installed or retrofitted with medical devices which are commonly used for subcutaneous medical procedures, such as catheters, guidewires, and biopsy needles. For example, the medical device 300 can be a separate component that is attached or retrofitted to a catheter, and can be positioned within the catheter or attached externally to the catheter.

The materials described for the medical devices are representative of a variety of materials which may be suitable for the medical devices, and any materials which are known in the art and possess the requisite characteristics are included in the scope of this disclosure.

The devices described herein can be constructed in a variety of methods. In one example, the echogenic subregions can be created by layering and creating holes in an intermediate layer (or external layer) through use of laser, EDM, or punching. Layers can have holes poked in them so as to expose underlying layers. In some examples, the subregions can be created by extrusion. In other examples, sandblasting or similar techniques can be used to create textured surfaces. A mask can be applied to a surface to create the desired arrangement of echogenic regions prior to adding texture.

While the disclosure has been illustrated and described in detail in the drawings and foregoing description, the same is to be considered as illustrative and not restrictive in character, it being understood that only the preferred embodiment has been shown and described and that all changes, equivalents, and modifications that come within the spirit of the following claims are desired to be protected. All publications, patents, and patent applications cited in this specification are herein incorporated by reference as if each individual publication, patent, or patent application were specifically and individually indicated to be incorporated by reference and set forth in its entirety herein. It will be understood that features or aspects discussed particularly in the context of one embodiment may be used with or incorporated in or with other features, aspects or embodiments.

## Claims

1. A medical device (100) detectable with ultrasound imaging equipment comprising:
an echogenic region (106), wherein the echogenic region includes a plurality of first subregions (108) being defined by borderlines and a plurality of second subregions (110), wherein the first subregions include an echogenic enhancement, and wherein the first subregions and second subregions are arranged in an alternating pattern wherein at least one second subregion lies between each pair of first subregions with no borderlines shared between any pair of the first subregions;
**characterized in that** the first subregions and second subregions are arranged in a checkerboard pattern, wherein a corner point of each of the first subregions is shared with one of the corner points of another first subregion.

2. The medical device of claim 1, wherein the medical device includes a planar structure which supports the echogenic region.

3. The medical device of claim 1, wherein the medical device includes a support structure having a cylindrically shaped surface, and wherein the echogenic region is positioned within the support structure.

4. The medical device of claim 1, wherein the first subregions include a metal film printed on a plastic surface.

5. The medical device of claim 1, further comprising a plastic structure, and wherein the echogenic enhancement includes a plurality of gaseous pits positioned within the plastic structure.

6. The medical device of claim 5, wherein the gaseous pits are formed by laser pitting.

7. The medical device of claim 1, wherein the echogenic enhancement includes a metal portion which is shaped to fit within a plastic receptacle of the medical device.

8. The medical device of claim 7, wherein the metal portion is formed by casting.

9. The medical device of claim 1, wherein the first subregions have a dimension between 0.5 mm and 2.5 mm.

10. The medical device of claim 1, wherein the echogenic region has a dimension greater than 2 mm.

11. The medical device of claim 1 configured to be placed subcutaneously within a body, wherein the medical device includes a tip portion having an outer wall that is substantially echolucent when positioned within a body;
wherein the first subregions include at least one compartment integrally located within at least the tip portion of the medical device adapted to house a fluid contrast agent; and
a fluid contrast agent within the at least one compartment, wherein the fluid contrast agent has an acoustic impedance and the tip portion has an acoustic impedance, wherein the acoustic impedance of the fluid contrast agent differs substantially from the acoustic impedance of the structure, so that when the medical device is positioned within a body the fluid contrast agent within the at least one compartment is imageable with ultrasonic imaging equipment.

12. The medical device of claim 11, wherein the fluid contrast agent includes microbubbles.

13. The medical device of claim 1 configured to be placed subcutaneously within a body, wherein the first subregions have at least one compartment adapted to receive a fluid contrast agent;
a fluid contrast agent within at least part of the at least one compartment, the fluid contrast agent including microbubbles configured to reflect harmonic echoes from an emitted linear ultrasound signal.

## Patentansprüche

1. Eine medizinische Vorrichtung (100), die mit Ultraschall-Bildgebungsausrüstung detektierbar ist, beinhaltend:
einen echogenen Bereich (106), wobei der echogene Bereich eine Vielzahl von ersten Teilbereichen (108), die durch Grenzlinien definiert sind, und eine Vielzahl von zweiten Teilbereichen (110) umfasst, wobei die ersten Teilbereiche eine echogene Verstärkung umfassen, und wobei die ersten Teilbereiche und zweiten Teilbereiche in einem abwechselnden Muster angeordnet sind, wobei mindestens ein zweiter Teilbereich zwischen jedem Paar von ersten Teilbereichen liegt, ohne gemeinsame Grenzlinien zwischen einem beliebigen Paar der ersten Teilbereiche;
**dadurch gekennzeichnet, dass** die ersten Teilbereiche und zweiten Teilbereiche in einem Schachbrettmuster angeordnet sind, wobei ein Eckpunkt jedes der ersten Teilbereiche mit einem der Eckpunkte eines anderen ersten Teilbereichs gemein ist.

2. Medizinische Vorrichtung nach Anspruch 1, wobei die medizinische Vorrichtung eine planare Struktur umfasst, die den echogenen Bereich stützt.

3. Medizinische Vorrichtung nach Anspruch 1, wobei die medizinische Vorrichtung eine Stützstruktur mit einer zylindrisch gestalteten Oberfläche umfasst und wobei der echogene Bereich innerhalb der Stützstruktur positioniert ist.

4. Medizinische Vorrichtung nach Anspruch 1, wobei die ersten Teilbereiche einen auf einer Plastikoberfläche gedruckten Metallfilm umfassen.

5. Medizinische Vorrichtung nach Anspruch 1, die ferner eine Plastikstruktur beinhaltet und wobei die echogene Verstärkung eine Vielzahl von Gasgrübchen umfasst, die innerhalb der Plastikstruktur positioniert sind.

6. Medizinische Vorrichtung nach Anspruch 5, wobei die Gasgrübchen durch Laser-Grübchenbildung gebildet sind.

7. Medizinische Vorrichtung nach Anspruch 1, wobei die echogene Verstärkung einen Metallabschnitt umfasst, der so gestaltet ist, dass er in einen Plastikbehälter der medizinischen Vorrichtung passt.

8. Medizinische Vorrichtung nach Anspruch 7, wobei der Metallabschnitt durch Gießen gebildet ist.

9. Medizinische Vorrichtung nach Anspruch 1, wobei die ersten Teilbereiche eine Abmessung zwischen 0,5 mm und 2,5 mm aufweisen.

10. Medizinische Vorrichtung nach Anspruch 1, wobei der echogene Bereich eine Abmessung von mehr als 2 mm aufweist.

11. Medizinische Vorrichtung nach Anspruch 1, die zur subkutanen Platzierung innerhalb eines Körpers ausgelegt ist, wobei die medizinische Vorrichtung einen Spitzenabschnitt umfasst, der eine Außenwand aufweist, die, wenn sie innerhalb eines Körpers positioniert ist, im Wesentlichen echoluzent ist;
wobei die ersten Teilbereiche mindestens ein Abteil umfassen, das sich mindestens integriert innerhalb des Spitzenabschnitts der medizinischen Vorrichtung befindet, das zum Aufnehmen eines Fluidkontrastmittels angepasst ist; und
ein Fluidkontrastmittel innerhalb des mindestens eines Abteils, wobei das Fluidkontrastmittel eine akustische Impedanz aufweist und der Spitzenabschnitt eine akustische Impedanz aufweist, wobei die akustische Impedanz des Fluidkontrastmittels sich wesentlich von der akustischen Impedanz der Struktur unterscheidet, sodass, wenn die medizinische Vorrichtung innerhalb eines Körpers positioniert ist, das Fluidkontrastmittel innerhalb des mindestens einen Abteils mit Ultraschall-Bildgebungsausrüstung abgebildet werden kann.

12. Medizinische Vorrichtung nach Anspruch 11, wobei das Fluidkontrastmittel Mikrobläschen umfasst.

13. Medizinische Vorrichtung nach Anspruch 1, die zur subkutanen Platzierung innerhalb eines Körpers ausgelegt ist, wobei die ersten Teilbereiche mindestens ein Abteil aufweisen, das zum Aufnehmen eines Fluidkontrastmittels angepasst ist;
ein Fluidkontrastmittel innerhalb mindestens eines Teils des mindestens einen Abteils, wobei das Fluidkontrastmittel Mikrobläschen umfasst, die zum Reflektieren harmonischer Echos von einem ausgesendeten linearen Ultraschallsignal ausgelegt sind.

## Revendications

1. Dispositif médical (100) détectable avec un équipement d'imagerie par ultrasons, comprenant :
une région échogène (106), la région échogène comprenant une pluralité de premières sous-régions (108) définies par des limites et une pluralité de deuxièmes sous-régions (110), les premières sous-régions comprenant un renforcement d'échogénicité, et les premières sous-régions et les deuxièmes sous-régions étant disposées de façon alternée, au moins une deuxième sous-région se trouvant entre chaque paire de premières sous-régions, sans partage de limites entre une paire quelconque des premières sous-régions ;
**caractérisé en ce que** les premières sous-régions et les deuxièmes sous-régions sont disposées en damier, un point situé dans un coin de chacune des premières sous-régions étant partagé avec l'un des points situés dans un coin d'une autre première sous-région.

2. Dispositif médical selon la revendication 1, le dispositif médical comprenant une structure plane qui supporte la région échogène.

3. Dispositif médical selon la revendication 1, le dispositif médical comprenant une structure de support ayant une surface de forme cylindrique, et dans lequel la région échogène est positionnée à l'intérieur de la structure de support.

4. Dispositif médical selon la revendication 1, dans lequel les premières sous-régions comprennent un film métallique imprimé sur une surface en plastique.

5. Dispositif médical selon la revendication 1, comprenant en outre une structure en plastique, et dans lequel le renforcement d'échogénicité comprend une pluralité de cavités gazeuses positionnées à l'intérieur de la structure en plastique.

6. Dispositif médical selon la revendication 5, dans lequel les cavités gazeuses sont formées par un procédé de formation de cavités au laser.

7. Dispositif médical selon la revendication 1, dans lequel le renforcement d'échogénicité comprend une partie métallique dont la forme est étudiée pour une insertion dans un réceptacle en plastique du dispositif médical.

8. Dispositif médical selon la revendication 7, dans lequel la partie métallique est formée par coulée.

9. Dispositif médical selon la revendication 1, dans lequel les premières sous-régions ont une dimension de 0,5 mm à 2,5 mm.

10. Dispositif médical selon la revendication 1, dans lequel la région échogène a une dimension supérieure à 2 mm.

11. Dispositif médical selon la revendication 1, configuré pour être placé par voie sous-cutanée à l'intérieur d'un corps, le dispositif médical comprenant une partie d'extrémité comportant une paroi extérieure qui est sensiblement anéchogène lorsqu'elle est positionnée à l'intérieur d'un corps ;
dans lequel les premières sous-régions comprennent au moins un compartiment intégré dans au moins la partie d'extrémité du dispositif médical, conçu pour contenir un agent de contraste fluide ; et
un agent de contraste fluide à l'intérieur dudit au moins un compartiment, l'agent de contraste fluide ayant une impédance acoustique et la partie d'extrémité ayant une impédance acoustique, l'impédance acoustique de l'agent de contraste fluide étant sensiblement différente de l'impédance acoustique de la structure, de telle sorte que lorsque le dispositif médical est positionné à l'intérieur d'un corps, l'agent de contraste fluide présent à l'intérieur dudit au moins un compartiment permet d'obtenir des images avec l'équipement d'imagerie par ultrasons.

12. Dispositif médical selon la revendication 11, dans lequel l'agent de contraste fluide comprend des microbulles.

13. Dispositif médical selon la revendication 1, configuré pour être placé par voie sous-cutanée à l'intérieur d'un corps, dans lequel les premières sous-régions comportent au moins un compartiment conçu pour recevoir un agent de contraste fluide ;
un agent de contraste fluide à l'intérieur d'au moins une partie dudit au moins un compartiment, l'agent de contraste fluide comprenant des microbulles configurées pour refléter les échos harmoniques d'un signal ultrasonore linéaire émis.
